(19) Europäisches Patentamt
European Patent Office
Office européen des brevets

(11) Veröffentlichungsnummer: **0 116 955**
**B1**

(12) # EUROPÄISCHE PATENTSCHRIFT

(45) Veröffentlichungstag der Patentschrift: **20.05.87**

(51) Int. Cl.[4]: **C 07 F 15/00**, C 07 C 91/30, C 07 C 93/14, A 61 K 31/28

(21) Anmeldenummer: **84101581.1**

(22) Anmeldetag: **16.02.84**

(54) **(1,2-Diphenyl-ethylendiamin)-platin(II)-Komplexverbindungen.**

(30) Priorität: **18.02.83 DE 3305636**

(43) Veröffentlichungstag der Anmeldung: **29.08.84 Patentblatt 84/35**

(45) Bekanntmachung des Hinweises auf die Patenterteilung: **20.05.87 Patentblatt 87/21**

(84) Benannte Vertragsstaaten: **AT BE CH DE FR GB IT LI NL SE**

(56) Entgegenhaltungen: **EP-A-0 055 300**

**CHEMICAL ABSTRACTS, Band 96, Nr. 23, 7. Juni 1982, Seite 83, Nr. 193435j, Columbus, Ohio, USA;**

(73) Patentinhaber: **Degussa Aktiengesellschaft, Weissfrauenstrasse 9, D-6000 Frankfurt am Main 1 (DE)**

(72) Erfinder: **Schönenberger, Helmut, Prof. Dr., Ahornstrasse 14, D-8401 Pentling (DE)**
Erfinder: **Wappes, Beate, Dr., Rüdigerstrasse 4, D-8400 Regensburg (DE)**
Erfinder: **Jennerwein, Margaretha, Brücklmaierweg 22, D-8400 Regensburg (DE)**
Erfinder: **von Angerer, Erwin, Dr., Karl- Esser-Strasse 3, D-8400 Regensburg (DE)**
Erfinder: **Engel, Jürgen, Dr., Cranachstrasse 25, D-8755 Alzenau (DE)**

**EP 0 116 955 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

In der japanischen Publikationsschrift 81 103 192 (Chemical Abstracts, Selects-Antitumor Agents, Ausgabe 13, 1982, Seite 10, 96:193435j) werden Stilbendiamin-Platin-Komplexe der folgenden Formel

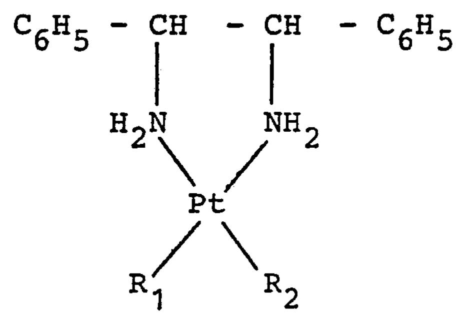

beschrieben. In der vorstehenden Formel bedeuten die Reste $R_1$ und $R_2$ Halogen, $NO_3$, $SO_4$, OH oder den Glucuronsäurerest. Für diese Verbindungen wird eine Antitumorwirkung angegeben.

Die Erfindung betrifft (1,2-Diphenyl-ethylendiamin)-platin(II)-Komplexverbindungen der allgemeinen Formel

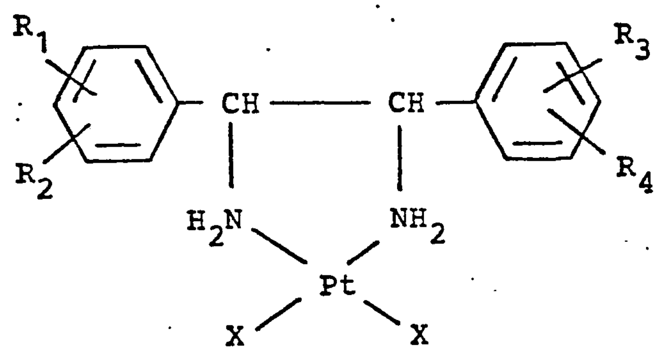

I

worin die Reste $R_1$, $R_2$, $R_3$ und $R_4$ gleich oder verschieden sind und Wasserstoff, Hydroxygruppen, $C_1$-$C_6$-Alkoxygruppen, gegebenenfalls durch Halogenatome oder $C_1$-$C_4$-Alkansulfonyloxygruppen substituierte $C_2$-$C_6$-Alkanoyloxygruppen oder $C_3$-$C_6$-Alkenoyloxygruppen bedeuten, wobei mindestens einer der Reste $R_1$, $R_2$, $R_3$ oder $R_4$ kein Wasserstoffatom ist, und X für das Äquivalent eines physiologisch verträglichen Anions steht, die als Arzneimittelwirkstoffe geeignet sind.

Die neuen erfindungsgemäßen Verbindungen besitzen eine ausgeprägte tumorhemmende Wirkung bei guter Verträglichkeit. Die tumorhemmende Wirkung zeigt sich insbesondere bei folgenden Tier- und Zellkulturmodellen: Leukämie (zum Beispiel Leukämie L 5222 der Ratte), Plasmazelltumore (zum Beispiel Plasmacytom ADJ/PC 6 der Maus), hormonabhängige Tumore (DMBA-induziertes und NMU-induziertes Mammacarcinom der Ratte, menschliche MCF 7-Mammacarcinom).

Darüberhinaus besitzen sie auch eine cytostatische Wirkung an hormonunabhängigen Mammacarcinom-Zellen (MDA-MB 231).

Im Vergleich zu den aus der japanischen Publikationsschrift 81 103 192 bekannten Verbindungen sind die erfindungsgemäßen Verbindungen beispielsweise stärker tumorhemmend wirksam bei geringerer Toxizität.

Im Vergleich zu dem bekannten antitumorwirksamen Wirstoff Cisplatin (cis-Dichloro-diamin-platin(II)) besitzen die erfindungsgemäßen Verbindungen eine geringere Toxizität, insbesondere eine niedrigere Nierentoxizität. Dies folgt aus Untersuchungen des Blutbildes, der Blutharnstoffkonzentration und der Nierenhistologie entsprechend behandelter Tiere (Mäuse). Dasselbe gilt beispielsweise auch hinsichtlich von Schädigungen des Darmepithels. Ebenfalls besitzen die erfindungsgemäßen Verbindungen eine außerordentlich geringe Knochenmarktoxizität.

Die folgenden Angaben betreffen bevorzugte Ausgestaltungen der Erfindung.

Die $C_1$-$C_6$-Alkoxygruppen und die gegebenenfalls wie angegeben substituierten $C_2$-$C_6$-Alkanoyloxygruppen können gerade oder verzweigt sein und bestehen im Falle der Alkoxygruppen vorzugsweise aus 1 bis 4 C-Atomen, im Falle der $C_2$-$C_6$-Alkanoyloxygruppen aus 2 bis 4 C-Atomen. Die $C_3$-$C_6$-Alkenoyloxygruppen können

ebenfalls gerade oder verzweigt sein und bestehen insbesondere aus 3 oder 4 C-Atomen. Als Halogensubstituenten kommen insbesondere Brom, Chlor und/oder Fluor in Frage. Die Alkanoyloxygruppen können einen oder mehrere (zum Beispiel 1 bis 6, insbesondere 1 bis 3) gleiche oder verschiedene Halogenatome enthalten. Insbesondere befinden sich 1, 2 oder 3 Halogenatome an einem C-Atom vorzugsweise an dem α-C-Atom. Ferner können sich die Halogenatome sowie der Alkansulfonyloxyrest vorzugsweise in β-Stellung des Alkanoyloxyrestes befinden. Beispielsweise handelt es sich um den Methan- oder Ethansulfonyloxyrest. Beispiele für Verbindungen der Formel I:

$R_1$ hat die angegebenen Bedeutungen (insbesondere OH) und steht in 2-, 3- oder 4-Stellung des Phenylringes, während $R_2$, $R_3$ und $R_4$ Wasserstoff bedeuten; $R_1$ und $R_3$ haben die angegebenen Bedeutungen (insbesondere OH) und stehen in 2-, 3- beziehungsweise 4-Stellung, während $R_2$ und $R_4$ Wasserstoff bedeuten; $R_1$ und $R_2$ sowie $R_3$ und $R_4$ haben die angegebenen Bedeutungen (insbesondere OH) und befinden sich vorzugsweise jeweils in 3- und 4-Stellung der beiden Phenylringe; $R_1$ und $R_2$ befinden sich vorzugsweise in 3- und 4-Stellung, während $R_3$ und $R_4$ Wasserstoff bedeuten.

Beispiele für die einzelnen Bedeutungen der Reste $R_1$, $R_2$, $R_3$ und $R_4$: Hydroxy, Methoxy, Acetoxy, Propionyloxy, Brom-, Chlor- oder Fluoracetoxy, β-Brom, β-Chlor- oder β-Fluorpropionyloxy, Dichlorbeziehungsweise Difluoracetoxy, Trichlor- beziehungsweise Trifluoracetoxy, Acryloyloxy.

Insbesondere kommen Verbindungen der Formel I in Frage, wo beide Phenylringe die gleichen Substituenten in den gleichen Positionen enthalten oder solche, wo nur der eine Phenylring einen oder zwei der angegebenen Substituenten enthält. Verbindungen mit besonders günstigen Eigenschaften sind zum Beispiel solche, wo beide Phenylringe in 4-Stellung oder in 3-Stellung jeweils eine Hydroxygruppe enthalten (1,2-bis-(4-hydroxy-phenyl-ethylendiamin- oder 1,2-bis-(3-hydroxy-phenyl-ethylendiamin-Derivate) und zwar sowohl in Form der Racemate als auch der Enantiomeren.

Die Reste X stellen die bekannten und üblichen physiologisch verträglichen und pharmazeutisch verwendbaren Anionen ein- oder mehrwertiger Säuren dar. Insbesondere kommen beispielsweise die Anionen folgender Säuren in Frage: HBr, HCl, HJ, $HNO_3$, $H_2SO_4$ ($SO_4^{--}$); $H_3PO_4$ ($HPO_3^{-}$); Kampfersulfonsäure, aliphatische oder aromatische Sulfonsäuren, beispielsweise $C_1$-$C_6$-Alkylsulfonsäuren (zum Beispiel Methansulfonsäure, Ethan-, Propan- oder Hexansulfonsäure), Benzol- oder Naphthalinsulfonsäure, die gegebenenfalls ein- oder zweifach durch Methylgruppen substituiert sind (Toluolsulfonsäure, insbesondere o- oder p-Toluolsulfonsäure); aliphatische $C_2$-$C_4$-Monocarbonsäuren, die gegebenenfalls ein-, zwei- oder dreifach durch Halogenatome (insbesondere Cl, F) substituiert sind (zum Beispiel Essigsäure, Propionsäure, Chloressigsäure, Dichloressigsäure, Trifluoressigsäure, Trichloressigsäure); aliphatische $C_2$-$C_{11}$-Dicarbonsäuren, die gegebenenfalls eine Doppelbindung enthalten (zum Beispiei Oxalsäure, Malonsäure, Malonsäure, welche in 2-Stellung durch eine oder zwei $C_1$-$C_4$-Alkylgruppen substituiert ist, Maleinsäure, Fumarsäure, Bernsteinsäure); aliphatische Monohydroxy- und Dihydroxy-Monocarbonsäuren mit 2 bis 6, insbesondere 2 bis 3 Kohlenstoffatomen, wobei es sich vorzugsweise um α-Monohydroxycarbonsäuren handelt wie Milchsäure, Glycerinsäure oder Glykolsäure; aliphatische Monohydroxy- und Dihydroxy-Di- und Tricarbonsäuren mit 3 bis 8 Kohlenstoffatomen, insbesondere 3 bis 6 Kohlenstoffatomen wie Tartronsäure, Äpfelsäure, Weinsäure, Malonsäure, die an dem mittelständigen C-Atom durch eine Hydroxygruppe und gegebenenfalls eine $C_1$-$C_4$-Alkylgruppe substituiert ist, Isozitronensäure oder Zitronensäure; Phthalsäure, die gebenenfalls durch eine Carboxygruppe (insbesondere in 4-Stellung) substituiert ist; Gluconsäure; Glucuronsäure; 1,1-Cyclobutandicarbonsäure; Organophosphorsäuren, wie Aldose- und Ketosephosphorsäuren (beispielsweise die entsprechenden Mono- und Diphosphorsäuren) zum Beispiel Aldose-6-phosphorsäuren wie D- oder L-Glucose-6-phosphorsäure, α-D-Glucose-1-phosphorsäure, D-Fructose-6-phosphorsäure, D-Galactose-6-phosphorsäure, D-Ribose-5-phosphorsäure, D-Fructose-1,6-diphosphorsäuren; Glycerinphosphorsäuren (wobei der Phosphorsäurerest an einem der endständigen oder an dem mittelständigen Glycerinsauerstoffatom gebunden ist) wie α-D,L-Glycerinphosphorsäure, β-Glycerinphosphorsäure; N-Phosphono-acetyl-Asparaginsäure (zum Beispiel L-Asparaginsäure).

Die Formel I umfasst auch die möglichen Enantiomere und Diastereomere. Falls die Verbindungen Racemate sind, können diese in an sich bekannter Weise, zum Beispiel mittels einer optisch aktiven Säure in die optisch aktiven Isomere gespalten werden. Es ist aber auch möglich, von vornherein enantiomere oder gegebenenfalls auch diastereomere Ausgangsstoffe einzusetzen, wobei dann als Endprodukt eine entsprechende reine optisch aktive beziehungsweise diastereomere Verbindung erhalten wird. Unabhängig von der Struktur der Reste X besitzt bereits der 1,2-Diphenyl-ethylendiamin-Teil 2 asymmetrische Kohlenstoffatome und kann daher in der Racemat-Form oder in links- beziehungsweise rechtsdrehender Form oder der meso-Form vorliegen. Zusätzliche Formen können durch verschiedene enantiomere beziehungsweise diastereomere Formen der Reste X entstehen. Besonders günstige Wirkungen besitzen die an beiden Asymmetriezentren des 1,2-Diphenyl-ethylendiamin-Teils gleich konfigurierten Komplexe.

Hinsichtlich des Platinatoms handelt es sich bei den erfindungsgemäßen Verbindungen der Formel I stets um die cis-Verbindungen.

Das Ausgangsamin II wird beispielsweise als Racemat, als Meso-Verbindung, als reine rechtsbeziehungsweise linksdrehende Form oder in einer sonstigen diastereomeren Form eingesetzt. Diese Konfiguration bleibt bei der Herstellung des Platinkomplexes erhalten. Besonders wirksam sind die Racemate der Formel I und deren optische Antipoden.

Das Verfahren zur Herstellung der erfindungsgemäßen Verbindungen I wird in einem Lösungsmittel bei Temperaturen zwischen 10 und 80°C, vorzugsweise 15 bis 50°C, insbesondere 18 bis 25°C durchgeführt. Als

Lösungsmittel kommen beispielsweise in Frage: Wasser, $C_1$-$C_6$-Alkanole (Methanol, Ethanol, tert.-Butanol), Tetrahydrofuran, Dioxan, Dimethylsulfoxid, Dimethylformamid, Ethylenglykoldimethylether, Diethylenglykoldimethylether sowie Gemische dieser Lösungsmittel, insbesondere Mischungen mit Wasser.

Die beiden Reaktionskomponenten (Platin-Verbindung und Verbindung II) werden vorzugsweise in äquimolaren Mengen eingesetzt. Der pH-Wert der Reaktionslösung soll zwischen 4 - 7, vorzugsweise im pH-Bereich 6 bis 7 liegen. Die Einstellung des pH-Wertes erfolgt insbesondere durch Zusatz von Alkali, vorzugsweise wässriger Natronlauge oder Kalilauge oder beispielsweise auch mittels Natriumcarbonat.

Als Tetrahalogen-platin(II)-Verbindungen (Säure sowie Alkalikomplexsalze) kommen die entsprechenden Tetrachloro-, Tetrabromo- und Tetrajodo-Verbindungen in Frage. Die Alkaliatome in den Alkali-tetrahalogeno-platin(II)-Komplexsalzen sind insbesondere Natrium und Kalium; es können aber auch die Lithium, Rubidium oder Cäsiumsalze verwendet werden.

Zweckmäßig wird das Diamin II in Form eines Säureadditionssalzes eingesetzt: zum Beispiel als Dihydrochlorid, Dihydrobromid, Dihydrojodid oder als Salz einer anderen Säure. Insbesondere kommen auch die Säuren in Frage, deren Anionen die Reste X bilden. Weiterhin kann das Diamin in Form des Acetats beziehungsweise Diacetats eingesetzt werden, wobei gegebenenfalls vor dem Mischen der Reaktionskomponenten Kaliumchlorid (beispielsweise 2 Mol pro 1 Mol Verbindung II) zugesetzt wird. Ebenso kann das Diamin II in Form des Carbonats eingesetzt werden.

In Verbindungen der Formel I können vorhandene freie phenolische Hydroxygruppen durch $C_2$-$C_6$-Alkanoylgruppen oder durch $C_3$-$C_6$-Alkenoylgruppen acyliert werden. Diese Alkanoyl- und Alkenoylgruppen können Halogenatome oder $C_1$-$C_4$-Alkansulfonyloxygruppen enthalten.

Diese Acylierung kann beispielsweise mittels $C_2$-$C_6$-Alkanoylhalogeniden beziehungsweise $C_3$-$C_6$-Alkenoylhalogeniden, die gegebenenfalls durch Halogenatome oder $C_1$-$C_4$-Alkansulfonylgruppen substituiert sind bei Temperaturen zwischen 10 und 80° C, insbesondere 20 - 30° C in Anwesenheit üblicher säurebindender Stoffe erfolgen. Insbesondere kommen als säurebindende Stoffe aliphatische tertiäre Amine wie zum Beispiel Diisopropylethylamin in Betracht.

Als inerte Lösungs- beziehungsweise Suspensionsmittel für die Acylierung kommen beispielsweise in Frage: niedere aliphatische Halogenkohlenwasserstoffe (Chloroform), aprotische Lösungsmittel wie Amide, $C_1$-$C_4$-Alkylamide und $C_1$-$C_4$-Dialkylamide aliphatischer $C_1$-$C_4$-Carbonsäuren (Dimethylformamid, Dimethylacetamid), N-Methyl-pyrrolidon, Dimethylsulfoxid oder Mischungen dieser Mittel. Man kann diese Acylierung zum Beispiel aber auch in einem Zweiphasensystem beispielsweise Wasser/Chloroform durchführen, wobei der unter Zuhilfenahme eines Anionenaustauschers gewonnene Dihydroxy-1,2-bis-(hydroxyphenyl) ethylendiamin-platin(II)-Komplex sich in der Wasserphase und das Gemisch von Säurechlorid und tertiärem Amin (Diisopropylethylamin) sich in der Chloroformphase befindet. Als Säurehalogenide kommen vorzugsweise die entsprechenden Chloride, Bromide und gegebenenfalls Jodide in Betracht.

Der Austausch der Liganden X gegen andere Liganden kann beispielsweise mittels der Silberhalogenidfällung erfolgen. Hierzu wird beispielsweise eine Dihalogeno-(1,2-diphenyl-ethylendiamin)-platin(II)-Verbindung der Formel I, worin X Halogen (Chlor, Brom oder Jod) bedeutet in einem Lösungs- oder Suspensionsmittel bei Temperaturen zwischen 10 bis 80° C, vorzugsweise 30 bis 50° C, insbesondere 35 bis 45° C mit den Silbersalzen einer anderen Säure, die der Bedeutung X entspricht, umgesetzt. Man kann hierbei aber auch als Silbersalz Silbernitrat (zum Beispiel wässrige Silbernitrat-Lösung) verwenden und erhält einen ionischen Diaquokomplex der Formel

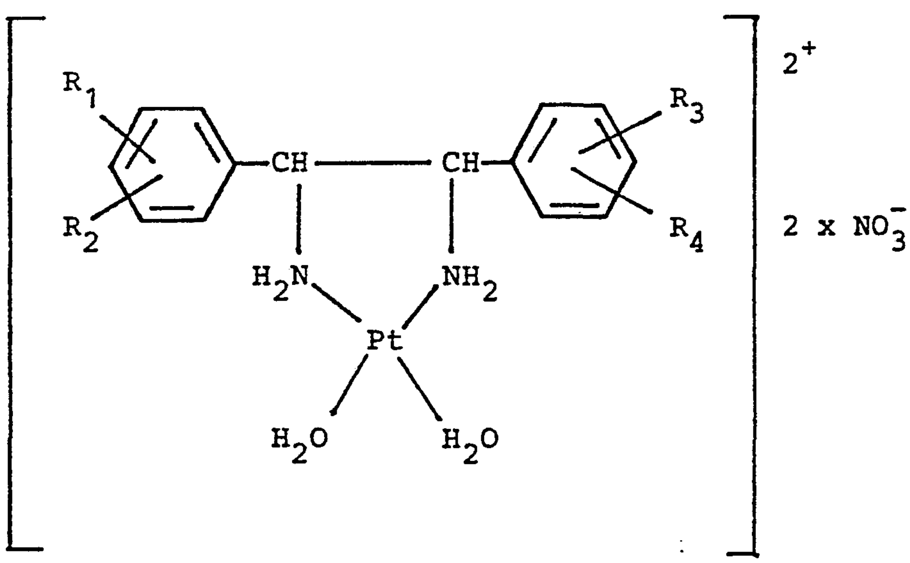

Aus diesem Komplex läßt sich der schwach gebundene Ligand Wasser leicht durch affinere Anionen (zum Beispiel $Cl^-$, $Br^-$ in Form von KCl, KBr, Malonat$^{2-}$, Cloracetat$^{(-)}$, Oxalat$^{2-}$, 1,1-Cyclobutandicarbonsäure-Anion$^{2-}$) sowie die übrigen angegebenen Säurereste X verdrängen.

Die gleichen Verbindungen lassen sich auch durch Umsetzung äquimolarer Mengen von HX und Nitratfreiem Platinkomplex (letzterer unter Verwendung von Anionenaustauschern in der Hydroxidform, zum Beispiel Dowex ® 1 - 8X) gewinnen.

Ein Austausch der Abgangsgruppe (zum Beispiel $SO_4^{2-}$- beziehungsweise Oxalatanion$^{2-}$) ist im Falle der Sulfato- beziehungsweise Oxalato-(1,2-diphenylethylendiamin)-platin(II)-Verbindungen auch durch Umsetzung mit Erdalkalisalzen, die den gewünschten X-Liganden (zum Beispiel Glycerinsäure) enthalten, möglich, sofern der entstehende Komplex wasserlöslich ist und damit die Abtrennung des schwer wasserlöslichen Erdalkalisulfats oder -oxalats erlaubt.

Für dieses Verfahren geeignete X-Liganden sind vorzugsweise Hydroxycarbonsäuren.

Die Lösungs- beziehungsweise Suspensionsmittel, die für das Herstellungsverfahren der Verbindungen I angegeben wurden, kommen auch für die Austauschreaktion in Frage (insbesondere eignen sich Wasser und Dimethylformamid sowie ferner Methanol, Ethanol, tert.-Butanol). Die Austauschreaktion wird beispielsweise in einem pH-Bereich zwischen 5,5 und 6 durchgeführt.

Die Herstellung des (±)-, (+)- sowie (-)-1,2-Bis-(4-methoxy-phenyl)-ethylendiamins aus der entsprechenden meso-Form ist in J.Med.Chem. 25, (1982), Seite 836 beschrieben. Die entsprechende meso-Form wird in Chemische Berichte 109 (1976), Seiten 1 - 40 (32) beschrieben. Die Herstellung der freien Hydroxyverbindungen erfolgt beispielsweise durch Etherspaltung mittels Bortribromid in Methylenchlorid bei -20 bis -80°C, vorzugsweise -60°C.

Die Herstellung anderer meso-Verbindungen der Formel II kann beispielsweise aus meso-1,2-Bis-(2-hydroxy-phenyl)ethylendiamin und den entsprechenden, durch Alkoxy substituierten Benzaldehyden

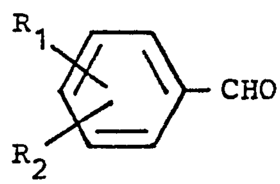

$R_1$ und $R_2$ = H oder $C_1$-$C_6$-Alkoxy

analog dem in Chemische Berichte 109 (1976), Seite 1 ff. beschriebenen Verfahren (Diaza-Cope-Umlagerung) erfolgen. Insbesondere eignet sich dieses Verfahren zur Herstellung von Verbindungen II, die in beiden Phenylringen gleichartig substituiert sind. Überführung der meso-Form in das Racemat und Trennung in die optischen Isomeren kann analog J.Med.Chem. 25 (1982), Seite 836 erfolgen.

Weiterhin können Ausgangsstoffe der Formel II (insbesondere unsymmetrisch substituierte) auf folgendem Weg erhalten werden:

Als Ausgangsmaterial dienen Stilbene, die an den beiden Phenylringen die Reste $R_1$, $R_2$, $R_3$ und $R_4$ enthalten und zum Beispiel durch Kupplung von Benzaldehyd, der durch die Reste $R_1$ und $R_2$ substituiert ist mit Titantetrachlorid analog der in Chemistry Letters 1973, Seiten 1041 - 1044 (published by the Chemical Society of Japan) beschriebenen Methode erhalten werden können. Derartige Stilbene können aber auch durch Umsetzung von Benzylmagnesiumhalogeniden, welche die Reste $R_1$ und $R_2$ enthalten mit unsubstituierten oder ringsubstituierten Benzaldehyden (das heißt Benzaldehyde, die die Reste $R_3$ und $R_4$ enthalten) analog der Methode erhalten werden, die in Berichte der Deutschen Chemischen Gesellschaft 37 (1904) Seiten 453 - 458 beschrieben wird. Durch Addition von N,N-Dichlorurethan an die Stilbendoppelbindung entstehen 1-Chlor-2-ethoxycarbamoyl-1,2-diphenylethane (mit den Resten $R_1$, $R_2$, $R_3$ und $R_4$ an den beiden Phenylringen), die sich basenkatalysiert zu 2,3-Diarylaziridinen cyclisieren lassen (analog der Methode, die in J.Org.Chemistry, Band 32 (1967), Seiten 75-78 und Band 31 (1966), Seiten 3625-3632 beschrieben wird). Produktgemische von cis- und trans-Aziridinen sind durch Chromatographie zu trennen. Aus diesen Aziridinen kann man die entsprechend substituierten 1,2-Diphenylethylendiamine beispielsweise dann wie folgt erhalten:

Der Aziridinring wird durch Erhitzen (80 - 120°C) mit Natrium-azid in einem Lösungsmittel (niedere Alkohole, gegebenenfalls im Gemisch mit Wasser) zum 1-Azido-2-amino-1,2-diphenylethan geöffnet. Bei dieser Reaktion entsteht aus dem Aziridin mit cis-ständigen Arylringen sterospezifisch das threo-konfigurierte Produkt, beziehungsweise aus trans-Aziridin das Produkt mit erythro-Konfiguration. Die Azidogruppe läßt sich in üblicher Weise mit $LiAlH_4$ in Ether zum Amin reduzieren. Etherspaltung mit Bortribromid führt dann zu hydroxysubstituierten 1,2-Diphenylethylendiaminen.

Eine weitere Möglichkeit zur Darstellung der Aziridine ist die Umsetzung von die Reste $R_1$, $R_2$, $R_3$ und $R_4$ enthaltenden Desoxybenzoinen, zugänglich durch Friedel-Crafts-Acylierung, mit Hydroxylamin in der hierfür üblichen Weise und Reduktion des so erhaltenen Oxims mit $LiAlH_4$ in Tetrahydrofuran zum Aziridin analog der in Tetrahedron Band 24 (1968), Seiten 4605-4623 sowie 6177-6184 beschriebenen Weise.

Die Herstellung von Ausgangsstoffen II über die entsprechenden Stilbene und Aziridine kann beispielsweise analog der im folgenden angegebenen Verfahrensvorschriften erfolgen:

1. Stilbensynthese

29 g (0,2 mol) 4-Chlorbenzaldehyd werden in 300 ml absolutem Dioxan gelöst und bei ca. 10°C unter Stickstoff mit 33 ml $TiCl_4$ versetzt. Zur gelben Suspension werden 39 g Zinkstaub gegeben, worauf sich die Suspension schwarzlila färbt. Es wird 4 - 5 Stunden unter Rückfluß erhitzt und nach dem Abkühlen mit 10 %iger $K_2CO_3$-Lösung hydrolisiert und mit Ether extrahiert. Die organische Phase wird über $MgSO_4$ getrocknet, das Lösungsmittel abrotiert und das Stilben umkristallisiert. Ausbeute: 75 - 90 %.

2. Aziridinsynthese

Zur Lösung von 24,9 g (0,1 mol) 4,4'-Dichlorstilben in 50 ml absolutem Benzol werden unter Stickstoff bei 5 - 10°C langsam 16 g (0,1 mol) N,N-Dichlorurethan zugetropft. Anschließend läßt man über Nacht bei Raumtemperatur rühren. Bei 5 - 10°C wird mit 100 ml 20%iger $NaHSO_3$-Lösung hydrolysiert, mit Ether ausgeschüttelt, die organischen Phasen werden mit 20 % NaCl-Lösung gewaschen, getrocknet und das Lösungsmittel abrotiert. Das Produkt bleibt als öliger Rückstand, der aus Ethanol umkristallisiert wird.

Ausbeute: 68 %. [1-Chlor-2-ethoxycarbamoyl-1,2-bis-(4-chlorphenyl)-ethan]

0,1 mol des so erhaltenen β-Chlorocarbamats, gelöst in 100 ml 96 %igem Ethanol, werden zu einer Lösung von 30 g (0,5 mol) KOH in 235 ml 96 %igem Ethanol gegeben. Der Reaktionsansatz wird 4 Stunden bei 50°C gerührt. Man verdünnt mit dem doppelten Volumen Wasser, extrahiert mit Ether oder Methylenchlorid, trocknet die organische Phase und zieht das Lösungsmittel ab. Das Produktgemisch aus cis- und trans-Aziridin wird durch Chromatographie an Kieselgel und Benzol/Methylenchlorid als Eluationsmittel getrennt.

Ausbeute: 45 %. [2,3-Bis-(4-chlorphenyl)-aziridin]

3. Ringöffnung mit Azid

20 mmol Aziridin werden in 80 ml Ethanol gelöst und mit 5,2 g (80 mmol) $NaN_3$ und 4,3 g (80 mmol) $NH_4Cl$ in 27 ml Wasser versetzt und 14 - 18 Stunden unter Rückfluß erhitzt. Anschließend wird mit Wasser verdünnt und mit Ether oder Methylenchlorid ausgeschüttelt. Nach dem Trocknen und Abrotieren des Lösungsmittels bleibt das Produkt als kristalliner Rückstand der aus Petrolether umkristallisiert wird.

Ausbeute: 74 %. [1-Azido-2-amino-1,2-bis-(4-chlorphenyl)-ethan]

Ausgangsstoffe der Formel II, worin einer oder mehrere der Reste $R_1$, $R_2$, $R_3$ und $R_4$ eine Hydroxygruppe darstellen, können an der Hydroxygruppe durch eine $C_2$-$C_6$-Alkanoylgruppe oder $C_3$-$C_6$-Alkenoylgruppe, die gegebenenfalls wie angegeben substituiert sind, acyliert werden. Diese Acylierung kann in inerten Lösungs- beziehungsweise Suspensionsmitteln wie Dioxan, Dimethylformamid, Benzol, Toluol, bei Temperaturen zwischen 0 bis 200°C vorzugsweise 20 bis 150°C erfolgen. Als Acylierungsmittel kommen zum Beispiel in Betracht: Säurehalogenide (Chloride, Bromide, Jodide) oder Säureanhydride der entsprechenden aliphatischen Carbonsäuren mit 2 bis 6 C-Atomen (gegebenenfalls wie angegeben substituiert). Diese Acylierung kann gegebenenfalls unter Zusatz eines säurebindenden Mittels wie Alkalicarbonaten, Alkalihydroxyden, Alkalialkoholaten oder eines tertiären Amins, zum Beispiel Triäthylamin oder Diisopropylethylamin erfolgen.

Eine andere Möglichkeit ist die Acylierung der bei der Diaza-Cope-Umlagerung anfallenden Diimine, zum Beispiel des aus N,N'-Bis-(4-methoxybenzyliden)-1,2-bis(4-methoxyphenyl)ethylendiamin durch Etherspaltung mit $BBr_3$ erhaltenen geschützten 1,2-Bis-(4-hydroxyphenyl)ethylendiamins (beide Aminogruppen sind durch die 4-Methoxy-benzylidengruppe geschützt). Die Acylierung der 1,2-Bis (hydroxyphenyl)ethylendiamine mit Benzalschutzgruppen kann so erfolgen, wie vorstehend beschrieben ist. Als säurebindendes Mittel kann jetzt auch Pyridin verwendet werden, wobei Pyridin gleichzeitig auch als Lösungsmittel dienen kann.

Das als Schutzgruppe dienende Benzaldehydderivat wird anschließend durch saure Hydrolyse und Wasserdampfdestillation abgetrennt.

Die erfindungsgemäßen Verbindungen sind zur Herstellung pharmazeutischer Zusammensetzungen geeignet. Die pharmazeutischen Zusammensetzungen beziehungsweise Medikamente können eine oder mehrere der erfindungsgemäßen Verbindungen oder auch Mischungen derselben mit anderen pharmazeutisch wirksamen Stoffen enthalten. Zur Herstellung der pharmazeutischen Zubereitungen können die üblichen pharmazeutischen Träger- und Hilfsstoffe verwendet werden. Die Arzneimittel können zum Beispiel enteral, parenteral (zum Beispiel intravenös, intramuskulär, subkutan) oder oral angewendet werden. Beispielsweise kann die Verabreichung in Form von Tabletten, Kapseln, Pillen, Dragees oder Zäpfchen erfolgen. Als Liquida kommen zum Beispiel in Frage: ölige oder wässrige Lösungen oder Suspensionen (zum Beispiel in Sesam- oder Olivenöl), Emulsionen, injizierbare wässrige und ölige Lösungen oder Suspensionen. Weiterhin können beispielsweise Trockenampullen, welche als Wirkstoff die erfindungsgemäße Verbindung I enthalten, hergestellt werden, wobei vor Gebrauch der Inhalt solcher Trockenampullen zum Beispiel in physiologischer Kochsalzlösung oder Gemischen aus physiologischer Kochsalzlösung und beispielsweise Dimethylsulfoxid aufgelöst wird.

**Pharmakologische beziehungsweise pharmazeutische Angaben**

Die erfindungsgemäßen Verbindungen sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der

Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953), Seite 1 bis 39; Journal of Pharmaceutical Sciences, Band 52 (1963), Seite 918 u.ff., H.v.Czetsch-Lindenwald, Hilfsstoffe für Pharmazie und angrenzende Gebiete; Pharm. Ind., Heft 2, 1961, Seite 72 u.ff.; Dr. H. P. Fiedler, Lexikon der Hilfsstoffe für Pharmazie, Kosmetik und angrenzende Gebiete Cantor, KG. Aulendorf in Württemberg 1971.

Beispiele hierfür sind Gelatine, natürliche Zucker wie Rohrzucker oder Milchzucker, Lecithin, Pektin, Stärke (zum Beispiel Maisstärke), Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel Methyloxypropylcellulose), Stearate, Magnesium- und Calciumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnussöl, Rhizinusöl, Olivenöl, Sesamöl, Baumwollsaatöl, Maisöl, Weizenkeimöl, Sonnenblumensamenöl, Kabeljau-Leberöl, Mono-, Di- und Triglyceride von gesättigten Fettsäuren $C_{12}H_{24}O_2$ bis $C_{18}H_{36}O_2$ und deren Gemische), pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Benzylbenzoat, Dioxolane, Glyzerinformale, Tetrahydrofurfurylalkohol, Polyglykolether mit $C_1$-$C_{12}$-Alkoholen, Dimethylacetamid, Lactamide, Lactate, Ethylcarbonate, Silicone (insbesondere mittelviskose Dimethylpolysiloxane) Magnesiumcarbonat und ähnliche.

Als weitere Hilfsstoffe kommen auch Stoffe in Frage, die den Zerfall bewirken (sogenannte Sprengmittel) wie: quervernetztes Polyvinylpyrrolidon, Natriumcarboxymethylstärke, Natriumcarboxymethylcellulose oder mikrokristalline Cellulose. Ebenfalls können bekannte Hüllstoffe verwendet werden wie zum Beispiel: Polyacrylsäureester, Celluloseether und ähnliche.

Zur Herstellung von Lösungen kommen beispielsweise Wasser oder physiologisch verträgliche organische Lösungsmittel in Frage, wie zum Beispiel Ethanol, 1,2-Propylenglykol, Polyglykole und deren Derivate, Dimethylsulfoxyd, Fettalkohole, Triglyceride, Partialester des Glycerins, Paraffine und ähnliche.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage: Polyvinylpyrrolidon, Sorbitanfettsäureester wie Sorbitanttrioleat, Lecithin, Acacia, Tragacanth, polyoxyethyliertes Sorbitanmonooleat, polyoxyethylierte Fette, polyoxyethylierte Oleotriglyceride, linolisierte Oleotriglyceride, Polyethylenoxyd-Kondensationsprodukte von Fettalkoholen, Alkylphenolen oder Fettsäuren oder auch 1-Methyl-3-(2-hydroxyethyl)-imidazolidon-(2). Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt.

Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solchen die Ölsäurereste enthalten) mit Ethylenoxyd erhalten werden (zum Beispiel 40 Mol Ethylenoxyd pro Mol Glycerid).

Beispiele für Oleotriglyceride sind Olivenöl, Erdnussöl, Rhizinusöl, Sesamöl, Baumwollsaatöl, Maisöl (siehe auch Dr. H.P. Fiedler "Lexikon der Hilfsstoffe für Pharmazie, Kosmetik und angrenzende Gebiete" 1971, Seite 191 bis 195).

Darüberhinaus ist der Zusatz von Konservierungsmitteln, Stabilisatoren, Puffersubstanzen, zum Beispiel Calciumhydrogenphosphat, kolloidales Aluminiumhydroxyd, Geschmackskorrigentien, Antioxydantien und Komplexbildnern (zum Beispiel Ethylendiaminotetraessigsäure) und dergleichen möglich. Gegebenenfalls ist zur Stabillisierung des Wirkstoffmoleküls mit physiologisch verträglichen Säuren oder Puffern auf einen pH-Bereich von ca. 3 bis 7 einzustellen. Im allgemeinen wird ein möglichst neutraler bis schwach saurer (bis pH 5) pH-Wert bevorzugt.

Als Antioxydantien kommen beispielsweise Natriummetabisulfit, Ascorbinsäure, Gallussäure, Gallussäurealkylester, Butylhydroxyanisol, Nordihydroguajaretsäure, Tocopherole sowie Tocopherole + Synergisten (Stoffe die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.

Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid und Formalinderivate in Betracht.

Die pharmazeutische und galenische Handhabung der erfindungsgemäßen Verbindungen erfolgt nach den üblichen Standardmethoden. Beispielsweise werden Wirkstoff(e) und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80°C, vorzugsweise 20 bis 50°C, insbesondere bei Raumtemperatur gearbeitet wird. Im übrigen wird auf das folgende Standardwerk verwiesen: Sucker, Fuchs,

Speiser, Pharmazeutische Technologie, Thieme-Verlag Stuttgart, 1978.

Die Applikation der Wirkstoffe beziehungsweise der Arzneimittel kann auf die Haut oder Schleimhaut oder in das Körperinnere erfolgen, beispielsweise oral, enteral, pulmonal, rectal, nasal, vaginal, lingual, intravenös, intraarteriell, intrakardial, intramuskulär, intraperitoneal, intracutan, subcutan.

Bei den parenteralen Zubereitungsformen handelt es sich insbesondere um sterile beziehungsweise sterilisierte Erzeugnisse.

Die erfindungsgemäßen Verbindungen zeigen in vivo am ADJ/PC 6 Plasmacytom und an der Leukämie L 5222 ebenso wie in vitro an der MDA-MB 231 und MCF-7 Mammatumor-Zellinie eine gute Antitumorwirkung. Beispielsweise wird am ADJ/PC 6 Plasmacytom der Balb/C-Maus bei einer Dosis von 3 x 20 mg/kg Maus ein % T/C-Wert von "Null" erreicht, das heißt es konnte sich bei keinem der Tiere ein Tumor entwickeln. Bei der Rattenleukämie L 5222 wurde in einer Dosierung von 3 x 20 mg/kg Ratte ein Anstieg der Überlebenszeit auf 175 %ILS erreicht. An der MDA-MB 231 Zellinie wurde in einer Konzentration von 1 x $10^{-5}$ Mol/l eine 90 %ige Hemmung des [$^3$H]-Thymidineinbaus erzielt, an der MCF-7 Zellinie bei gleicher Konzentration eine 95 %ige Hemmung.

Diese Antitumorwirkung ist mit der Wirkung des bekannten Arzneimittels Cisplatin vergleichbar beziehungsweise besser. Die niedrigste, bereits antitumoral wirksame Dosis in den oben angegebenen Tierversuchen ist beispielsweise 3 x 10 mg/kg ip (= intraperitoneal) (ADJ/PC 6) und ca. 3 x 15 mg/kg ip (L 5222). Als allgemeiner Dosisbereich für die Wirkung kommt beispielsweise in Frage: 3 x 20 mg - 3 x 30 mg/kg ip.

Indikationen, für die die erfindungsgemäßen Verbindungen in Betracht kommen können:

Bronchial-, Testes-, Ovarial-, Cervix-, Prostata-, EEndometrium-, Blasencarcinome, Melanom, Carcinome im Hals-Kopf-Bereich.

Kontraindikationen:

Schwangerschaft, schwere Knochenmarksdepression.

Die neuen erfindungsgemäßen Verbindungen besitzen eine ausgeprägte tumorhemmende Wirkung bei guter Verträglichkeit. Die tumorhemmende Wirkung zeigt sich insbesondere bei folgenden Tier- und Zellkulturmodellen: Leukämie (zum Beispiel Leukämie L 5222 der Ratte und L 1210 der Maus); Plasmazelltumore (zum Beispiel ADJ/PC 6 Plasmacytom der Maus); hormonabhängige Tumore (B 16 Melanom der Maus, menschliche MCF7 Mammacarcinom Zellinie); Cytostatica-resistente Tumore (Cisplatin- und Daunomycinresistenter Ehrlich Ascites Tumor der Maus). Darüberhinaus besitzen sie auch eine cytostatische Wirkung an hormonunabhängigen Mammacarcinom-Zellen (MDA-MB 231).

Die pharmazeutischen Zubereitungen enthalten im allgemeinen zwischen 100 bis 200 mg, vorzugsweise 150 mg der erfindungsgemäßen aktiven Komponente(n).

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: ölige oder alkoholische beziehungsweise wässrige Lösungen sowie Suspensionen und Emulsionen. Bevorzugte anwendungsformen sind Tabletten, die zwischen 100 und 200 mg oder Lösungen, die zwischen 0,02 bis 0,04 % an aktiver Substanz enthalten.

Die Einzeldosis der erfindungsgemäßen aktiven Komponenten kann beispielsweise liegen

a) bei oralen Arzneiformen zwischen 100 und 200 mg vorzugsweise 150 mg,

b) bei parenteralen Arzneiformen (als intravenöse Dauerinfusion) zwischen 100 und 200 mg/m$^2$, vorzugsweise 150 mg/m$^2$,

c) bei Arzneiformen zur lokalen Applikation auf die Haut und Schleimhäute (zum Beispiel in Form von Lösungen, Lotionen, Emulsionen, Salben und so weiter) zwischen 1 und 5 %, vorzugsweise 2,5 %.

Beispielsweise können 3 mal täglich 1 bis 4 Tabletten mit einem Gehalt von 100 bis 200 mg wirksamer Substanz oder zum Beispiel bei intravenöser Injektion eine Dauerinfusion von 1000 ml Inhalt mit 100 bis 200 mg Substanz/m$^2$ Körperoberfläche empfohlen werden. Bei oraler Verabreichung ist die minimale tägliche Dosis beispielsweise 300 mg; die maximale tägliche Dosis bei oraler Verabreichung soll nicht über 800 mg liegen.

Die akute Toxizität der erfindungsgemäßen Verbindungen an der Maus (ausgedrückt durch LD 50 mg/kg; Methode nach Miller und Tainter: Proc. Soc. Exper. Biol. a. Med. 57 (1944) 261) liegt beispielsweise bei ip Applikation oberhalb 120 mg/kg.

Die Arzneimittel können in der Humanmedizin verwendet werden.

**Beispiel 1**

(+)-Dichloro-[1,2-bis-(4-hydroxy-phenyl)-ethylendiamin]-platin(II)

Zur Lösung von 830 mg (2 mmol) $K_2PtCl_4$ in 6 ml $H_2O$ werden 2 mmol (+)-1,2-Bis-(4-hydroxy-phenyl)-ethylendiamin-dihydrobromid, gelöst in 4 ml Wasser, zugetropft. Die Lösung wird mit 0,5 n NaOH neutralisiert, wobei sich ein gelber Niederschlag bildet. Man läßt unter Lichtausschluß bei Raumtemperatur rühren und neutralisiert in Abständen von 1 - 2 Stunden. Nach 9 - 10 Stunden wird abgesaugt, mit Wasser chloridfrei gewaschen und getrocknet. Die Mutterlauge wird weiter gerührt und mehrmals neutralisiert. Nach ca. 4 Tagen zeigt die Konstanz des pH-Wertes das Ende der Reaktion an. Man saugt nochmals ab und behandelt den Niederschlag wie oben angegeben. Das so erhaltene gelbe Pulver ist sehr dielektrisch. (Ausbeute: 94 %)

Reinigung:

1020 mg (2 mmol) des gefällten Komplexes werden fein zermörsert, in 150 ml $H_2O$ aufgeschlämmt und mit 697 mg (4,1 mmol) Silbernitrat (gelöst in 40 ml Wasser) 15 Stunden bei Raumtemperatur gerührt, wobei der entsprechende Nitratokomplex entsteht. Das gebildete Silberhalogenid wird abzentrifugiert. Aus der überstehenden Lösung werden mit 0,5 n Salzsäure überschüssige Silberionen ausgefällt und nochmals zentrifugiert. Die Lösung des Nitratokomplexes wird abgetrennt, mit 8 mmol Kaliumchlorid (gelöst in 5 ml Wasser) versetzt, neutralisiert und einige Stunden gerührt. Der gereinigte Komplex wird abgesaugt, gewaschen und getrocknet. F: 340 - 350°C (unter Zersetzung). Ausbeute: 50 %.

IR-Spektrum in KBr: (= scharfe Bande, mittlere Bande)

3260 s, 3195 s (NH), 1620 s, 1600 s, 1520 s (NH), 1250 s, 1180 s, 830 s, 810 m, 770 m, 565 m, 530 m (PtN), 320 m (PtCl)

(-)- und (±)-Dichloro-[1,2-bis-(4-hydroxy-phenyl)-ethylendiamin]-platin(II) werden analog hergestellt.

(±)-Dichloro-[1,2-bis(4-hydroxy-phenyl)-ethylendiamin]-platin(II)

F.: 355 - 358°C (unter Zersetzung)

Ausbeute: 88 %, gelbes Pulver

IR-Spektrum in KBr:

3260 s, 3195 (NH), 1620 s, 1520 s, (NH), 1450 m, 1260 s, 1240 m, 1180 s, 830 s, 810 s, 765 m, 610 m, 570 m (PtN), 320 m (PtCl)

(-)-Dichloro-[1,2-bis-(4-hydroxy-phenyl)-ethylendiamin]-platin(II)

F.: 340 - 350°C (unter Zersetzung)

IR-Spektrum: siehe (+)-Dichloro-[1,2-bis-(4-hydroxy-phenyl)-ethylendiamin]-platin(II).

Herstellung der Ausgangsstoffe II:

Die Herstellung der entsprechenden (±)-, (+)- und (-)-Methylether ist in J.Med.Chem. 25 (1982), Seite 836 angegeben. Die freien Hydroxy-Verbindungen können hieraus zum Beispiel durch Etherspaltung gemäß folgender Vorschrift erhalten werden:

3,54 g (13 mmol) (-)-1,2-Bis(p-methoxyphenyl)ethylendiamin werden in 130 ml absolutem Methylenchlorid gelöst und auf -60°C gekühlt. Bei dieser Temperatur gibt man 4,95 ml (53 mmol) Bortribromid zu und rührt 30 Minuten im Kühlbad. Anschließend läßt man auf Raumtemperatur erwärmen und über Nacht weiterrühren. Unter Eis-Kochsalz-Lösung wird mit 10 ml Methanol hydrolysiert und bis zur Trockene abrotiert. Zur Reinigung wird in Methanol aufgenommen und mit Ether ausgefällt.

Ausbeute: 3,3 g (63 %)

IR: 3100 s sehr breit, 2000 w breit (NH), 1640 s, 1605 s, 1545 s, 1520 s, 1505 s, 1290 s, 1260 s, 1215 s, 860 s, 770 m.

**Beispiel 2**

(-)-Sulfato-[1,2-bis-(4-hydroxy-phenyl)-ethylendiamin]-platin(II) x 2 $H_2O$

1020 mg (2 mmol) (-)-Dichloro-[1,2-bis-(4-hydroxy-phenyl)-ethylendiamin]-platin(II) werden fein verrieben und in 100 ml Wasser suspendiert. Die auf 40°C erwärmte Suspension wird mit 624 mg (2 mmol) $Ag_2SO_4$ (gelöst in 80 ml Wasser) versetzt und über Nacht unter Lichtausschluß gerührt. Anschließend wird filtriert, eine kleine Probe in der Kälte mit 0,1 n HCl auf Silberionen geprüft und die Lösung im Rotationsverdampfer auf 5 ml eingeengt. Der Niederschlag wird abgesaugt mit Eiswasser gewaschen und getrocknet.

Weißes Pulver: F.: Ca. 295°C (bei 250°C Verfärbung)

Die Verbindung enthält 2 Molekülteile Wasser.

Ausbeute: 30 %

IR-Spektrum in KBr:

3200 s breit, 1610 m, 1520 s, 1250 s, 1180 s, 1120 s, 1020 s, 830 m.

**Beispiel 3**

(-)-1,1-Cyclobutandicarboxylat-[1,2-bis-(4-hydroxy-phenyl)-ethylendiamin]-platin(II)

1020 mg (2 mmol) (-)-Dichloro-[1,2-bis-(4-hydroxy-phenyl)-ethylendiamin]-platin(II) werden fein vermahlen und in 150 ml Wasser suspendiert. Die auf 60°C erwärmte Suspension wird mit 645 mg (3,8 mmol) $AgNO_3$ (gelöst in 5 ml Wasser) versetzt und über Nacht bei Raumtemperatur unter Lichtausschluß gerührt. Nach Einengen im Rotationsverdampfer auf 30 ml wird filtriert und das Filtrat mit einer Lösung von 274 mg (1,9 mmol) 1,1-Cyclobutandicarbonsäure in 25 ml Wasser versetzt und anschließend langsam mit 0,1 n NaOH auf pH 5,5 - 6,0 gebracht. Nun wird auf ca. 15 ml eingeengt (im Rotationsverdampfer), abgesaugt, gewaschen und über $P_2O_5$ getrocknet.

Weißes Pulver: F.: ca. 303° C (Verfärbung ab 280° C).
Die Verbindung enthält 1 Molekül Wasser.
Ausbeute: 24 %
IR-Spektrum in KBr:
3100 s breit, 1630 s, 1640 s, 1400 s, 850 s.

**Beispiel 4**

meso-Dichloro-[1,2-bis-(3,4-dimethoxy-phenyl)-ethylen-diamin]-platin(II)

332 mg (1 mmol) 1,2-bis-(3,4-dimethoxy-phenyl)-ethylen-diamin werden in 150 ml heißer 0,02 N HCl gelöst. Die Lösung wird langsam unter Rühren zur Lösung von 415 mg $K_2PtCl_4$ in 5 ml $H_2O$ gegeben. Die Lösung wird mit 0,5 N NaOH neutralisiert und mit ca. 2 g KCl versetzt. Man läßt bei ca. 70° C unter Lichtausschluß rühren und neutralisiert in Abständen 1 - 2 Stunden. Nach 12 Stunden wird abgesaugt, mit 0,5 N HCl, $H_2O$ und Aceton gewaschen und getrocknet.

F. ca. 280° C (Zersetzung)
Ausbeute: 79 %, gelbes Pulver
IR-Spektrum in KBr:
3220 s, 3110 m (NH), 2920 m (CH), 1595 m, 1520 s, 1270 s, 1030 s, 805 m, 765 m, 535 w, 330 m (PtCl)

**Beispiel 5**

meso-Dichloro-[1,2-bis-(4-methoxy-phenyl)-ethylendiamin]-platin(II)

415 mg (1 mmol) $K_2PtCl_4$, gelöst in ca. 10 ml 40 %igem warmen t-Butanol werden mit 272 mg (1 mmol) 1,2-Bis(p-methoxy-phenyl)-ethylendiamin in 40 ml 50 %igem t-Butanol versetzt. Man rührt 2 Stunden bei 50° C unter Lichtausschluß. Das Produkt wird abgesaugt, mit Wasser und t-Butanol gewaschen und getrocknet.

F. ca. 220° C (Zersetzung)
Ausbeute: 84 %, hellgelbes Pulver
IR-Spektrum in KBr:
3240 m (NH), 1610 s, 1580 s, 1510 s (NH), 1460 m, 1250 s, 1180 s, 1030 s, 540 m, 315 m (PtCl)

Die folgenden Verbindungen wurden analog Beispiel 1 hergestellt.

**Beispiel 6**

(±)-Dichloro[l,2-bis-(3-methoxy-phenyl)-ethylendiamin]-platin(II)
F. ca. 335° C (Zersetzung)
Ausbeute: 83 %, gelbes Pulver
IR-Spektrum in KBr:
3270 s (NH), 2950 m (CH), 1600 s, 1300 s, 1235 s, 790 s, 700 s, 560 w, 465 w, 325 m (PtCl)

**Beispiel 7**

(±)-Dichloro-[1,2-bis-(3-hydroxy-phenyl)-ethylendiamin]-platin(II)
F. ca. 360° C (Zersetzung)
Ausbeute: 68 %, gelbes Pulver
IR-Spektrum in KBr:
3260 s, 3200 m (NH), 1600 s, 1465 s, 1220 s, 705 s, 470 m, 320 m (PtCl)

**Beispiel 8**

meso-Dichloro-[1,2-bis-(3-hydroxy-phenyl)-ethylendiamin]-platin(II)
F. ca. 275° C (Zersetzung)
Ausbeute: 53 %, hellgelbes Pulver
IR-Spektrum in KBr:
3200 s, 3110 m (NH), 1590 s, 1460 s, 1045 m, 780 s, 705 s, 535 w, 325 m (PtCl)

**Beispiel 9**

(±)-Dichloro-[1,2-bis(4-methoxy-phenyl)-ethylendiamin]-platin(II)
F. ca. 335°C (Zersetzung)
Ausbeute: 55 %, gelbes Pulver
IR-Spektrum in KBr:
3260 s, 2310 m, 3170 s (NH), 1610 s, 1580 m, 155 m (NH), 1460 s, 1250 s, 1180 s, 1040 s, 825 s, 530 m, 310 (PtCl)

**Beispiel 10**

threo-Dichloro-[1-(4-hydroxy-phenyl)-2-phenyl-ethylendiamin]-platin(II)
F. ca. 280°C (Zersetzung)
Ausbeute: 57 %, hellgelbes Pulver
IR-Spektrum in KBr:
3200 s, 3100 m (NH), 1610 m, 1570 m, 1500 s, 1175 s, 755 m, 700 m, 520 w, 325 m (PtCl)

**Beispiel 11**

threo-Dichloro-[1-(4-methoxy-phenyl)-2-phenyl-ethylendiamin]-platin(II)
F. ca. 280°C (Zersetzung)
Ausbeute: 57 %, hellgelbes Pulver
IR-Spektrum in KBr:
3200 s, 3100 s (NH), 1610 m, 1570 m, 1510 s, 1260 s, 1070 s, 710 s, 320 m (PtCl)

**Beispiel 12**

Dichloro-[1-(3,4-dimethoxy-phenyl)-2-phenyl-ethylendiamin]-platin(II)
F. ca. 335°C (Zersetzung)
Ausbeute: 85 %, gelbes Pulver
IR-Spektrum in KBr:
3240 s (NH), 1600 m, 1520 s, 1320 s, 1150 s, 1030 s, 770 m, 710 m, 520 w, 320 m (PtCl)

**Herstellung von Ausgangsstoffen für die Beispiele 4-12**

Die Ausgangsstoffe der Formel II wurden als Hydrobromide oder Hydrochloride eingesetzt. Die Hydrobromide der Hydroxyverbindungen werden nach der Etherspaltung mit Bortribromid isoliert. Zur Herstellung der Hydrochloride löst man die Basen der entsprechenden Verbindungen in Alkohol, leitet HCl ein und fällt das Produkt mit Ether aus.

Aus den Hydrobromiden können die Hydrochloride durch Umsetzen mit einem Ionenaustauscher, Abtrennen der wäßrigen Lösung und Abdampfen des Wassers hergestellt werden.

Allgemeines zur Synthese der Ausgangsstoffe II.

Die Ausgangsverbindungen für die Beispiele 4-9 wurden nach dem Verfahren hergestellt, welches in Chemische Berichte, 109 (1976), Seite 1 ff beschrieben wird. Die Etherspaltung erfolgte mit Bortribromid wie unter Beispiel 1 (bei Herstellung der Ausgangsstoffe II) angegeben wird. 1-(4-Methoxy-phenyl)-2-phenyl-ethylendiamin (Ausgangsstoff für Beispiel 11) wurde aus trans-4-Methoxy-stilben analog dem in der Anmeldung angegebenen Verfahren hergestellt. (siehe unter Stilbensynthese, Aziridinsynthese)

meso-1,2-Bis(3-methoxyphenyl)ethylendiamin
(F. 115-117°C)
Diese Verbindung läßt sich analog dem 1,2-Bis-(4-methoxyphenyl)-ethylendiamin nach der in J.Med.Chem. 25 (1982) Seite 836 ff. angegebenen Methode herstellen.

meso-N,N'-Bis(3-methoxybenzyliden)-1,2-bis(3-methoxyphenyl)-ethylendiamin
(F. 120°C aus Acetonitril)
Synthese analog J.Med.Chem. 25 (1982), Seite 836 ff.

(±)-1,2-Bis(3-methoxyphenyl)ethylendiamin
Das zuvor genannte Diimin wird geschmolzen und bei 150°C - 160°C ca. 15 Minuten gerührt. Nach abkühlen auf etwa 90°C wird mit 3N $H_2SO_4$ versetzt und einer Wasserdampfdestillation unterworfen. Die zurückbleibende Lösung wird heiß filtriert, bei 0 - 20°C auf pH 2 gebracht und mit Ethanol versetzt. Das

auskristallisierte Sulfat wird abgetrennt, mit NaOH die Base freigesetzt, mit Methylenchlorid/Chloroform ausgeschüttelt und einrotiert. Das Produkt bleibt als öliger Rückstand. (IR-Spektrum (Film): 3400 breit, 1600 s, 1500 s, 1270 s, 1050 m, 710 s) (s = scharfe Bande, m = mittlere Bande)

meso- und (±)-1,2-Bis(3,4-dimethoxyphenyl)ethylendiamin

Diese Verbindungen lassen sich analog den 4-mono-sub-stituierten Diaminen nach J.Med.Chem. 25 (1982), Seite 836 ff. herstellen.

F. der meso-Form 185,5 - 187° C (aus Chloroform)

F. der (±)-Form 82 - 83° C.

threo- und erythro-1-(4-Methoxyphenyl)-2-phenylethylen-diamin

Diese Verbindungen wurden aus 4-Methoxystilben nach dem in der Anmeldung angegebenen Verfahren und anschließender Reduktion des Azids mit $LiAlH_4$ in absolutem Diethylether erhalten. Zutropfen von 15,2 mmol Azid in 60 ml absolutem Ether zu der Suspension von 1,4 g $LiAlH_4$ in 70 ml absolutem Ether unter Eiskühlung, anschließendes Erhitzen unter Rückfluß (4,5 Stunden), Abkühlen und Hydrolyse mit Wasser.

4-Methoxystilben (F. 130 - 131° C)

19,6 g (0,1 mol) 4-Hydroxystilben, 135 g $K_2CO_3$ und 135 g Methyljodid werden in 500 ml Dimethylformamid 20 Stunden bei Raumtemperatur gerührt. Nach Verdünnen mit $H_2O$ wird mit Methylenchlorid ausgeschüttelt.

threo-1-(4-Methoxyphenyl)-2-phenylethylendiamin (Öl; IR-Spektrum (Film): 3390 m, 3310 m ($NH_2$), 1610 s, 1510 s, 1270 s, 1070 s, 1040 s);

erythro-1(4-Methoxyphenyl)-2-phenylethylendiamin (F. 90 - 91° C) und

1-(3,4-Dimethoxyphenyl)-2-phenylethylendiamin (Öl; IR-Spektrum (Film): 3380 m, 3310 m ($NH_2$), 2950 (CH aliphatisch), 1600 m, 1510 s, 1470 s, 1260 s, 1140 s, 1030 s) wurden aus den entsprechenden Aziridinen über Ringöffnung mit Azid und nachfolgender Reduktion mit $LiAlH_4$ dargestellt. (siehe unter Herstellung der Ausgangsstoffe) Beispielsweise wurde das 1-(3,4-Dimethoxyphenyl)-2-phenylethylendiamin aus 1-Oximino-1-(3,4-Dimethoxyphenyl)-2-phenyl-ethan über das 2-(3,4-Dimethoxy-phenyl)-3-phenyl-aziridin erhalten. Die Reduktion des Oxims mit $LiAlH_4$ wird wie folgt durchgeführt:

Zur Suspension von 760 mg (20 mmol) $LiAlH_4$ in 16 ml absolutem Tetrahydrofuran werden langsam 2,71 g (10 mmol) Oxim, gelöst in 70 ml absolutem Tetrahydrofuran, zugetropft. Anschließend wird 3 Stunden unter Rückfluß erhitzt. Unter Eiskühlung wird mit $H_2O$ hydrolysiert, vom Aluminiumhydroxid abgesaugt und mit Ether extrahiert. Nach dem Entfernen des Lösungsmittels bleibt das Aziridin als gelbes Öl.

Die freien Hydroxy-Verbindungen wurden aus den Methylethern durch Etherspaltung nach der in der Anmeldung beschriebenen Methode dargestellt:

(±)-1,2-Bis(3-hydroxyphenyl)ethylendiamin

IR-Spektrum (KBr): 3340 m, 3290 m (NH), 1610 s, 1460 s, 1160 m

meso-1,2-Bis(3-hydroxyphenyl)ethylendiamin

IR-Spektrum (KBr): 3370 m, 3340 m (NH), 1600 s, 1470 s, 1260 s

threo-1(4-Hydroxyphenyl)2-phenylethylendiamindihydro-bromid

IR-Spektrum (KBr): 3400 m, 3000 s breit (NH, OH), 1600 s, 1530 s, 720 s

erythro-1-(4-hydroxyphenyl)2-phenylethylendiamindihydro-bromid

IR-Spektrum (KBr): 3400 m, 3000 s breit (NH, OH), 1590 s, 1510 s, 1290 s, 1060 s

**Beispiele für pharmazeutische Zubereitungen**

**Beispiel für Injektionslösung**

In 800 ml Wasser für Injektionszwecke werden unter Rühren 9 g Natriumchlorid gelöst und der pH-Wert mit Hilfe von konzentrierter Salzsäure (38 %) auf 2,5-3,5 (vorzugsweise 3,0) eingestellt. Unter Rühren wird dann 1 g der Substanz (+)-Dichloro-[1,2-bis-(4-hydroxy-phenyl)-ethylendiamin]-platin II gelöst. Der pH-Wert wird kontrolliert und, falls erforderlich, erneut mit Salzsäure auf 2,5 - 3,5 eingestellt. Schließlich wird mit Wasser für Injektionszwecke das Volumen auf 1 Liter aufgefüllt und nochmals der pH-Wert überprüft.

Diese Lösung wird unter aseptischen Bedingungen über ein Membranfilter von 0,22 µm Porenweite sterilfiltriert und in 50 ml-Injektionsflaschen (braun) der hydrolytischen Klasse I zu 50 ml abgefüllt. Die Injektionsflaschen werden mit teflonbeschichteten Gummistopfen verschlossen und mit Aluminiumbördelkappen versehen. 1 ml Lösung enthält 1 mg Wirkstoff.

**Beispiel für Lyophilisat**

In 800 ml Wasser für Injektionszwecke werden unter Rühren 9 g Natriumchlorid und 10 g D-Mannit gelöst. Mit konzentrierter Salzsäure (38 %) stellt man einen pH-Wert von 2,5 - 3,5 (vorzugsweise 3,0) ein. In dieser Lösung wird unter Rühren 1 g der Substanz (+)-Dichloro-[1,2-bis-(4-hydroxy-phenyl)-ethylendiamin]-platin II gelöst. Der pH-Wert wird kontrolliert und, falls erforderlich, erneut mit Salzsäure auf 2,5 - 3,5 eingestellt.

Schließlich wird mit Wasser für Injektionszwecke das Volumen auf 1 Liter aufgefüllt und nochmals der pH-Wert überprüft.

Diese Lösung wird unter aseptischen Bedingungen über ein Membranfilter von 0,22 µm Porenweite sterilfiltriert und zu 10 ml in braune 15 ml Injektionsflaschen der hydrolytischen Klasse I abgefüllt. Diese Flaschen werden mit einem Gefriertrocknungsstopfen versehen und in einer geeigneten Anlage lyophilisiert. Nach der Trocknung begast man mit sterilem getrockneten Stickstoff und verschließt die Flaschen endgültig in der Anlage. Die Stopfen werden durch eine Bördelkappe gesichert.

Für die intravenöse Anwendung wird das Lyophilisat in 10 ml Wasser für Injektionszwecke rekonstituiert. 1 ml Lösung enthält 1 mg Wirkstoff.

**Patentansprüche**

1. (1,2-Diphenyl-ethylendiamin)-platin(II)-Komplexverbindungen der allgemeinen Formel

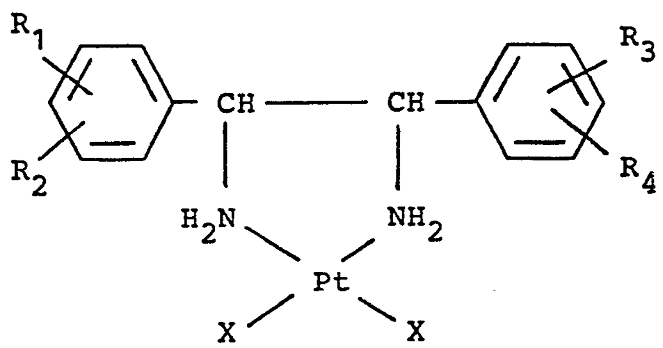

I

worin die Reste $R_1$, $R_2$, $R_3$ und $R_4$ gleich oder verschieden sind und Wasserstoff, Hydroxygruppen, $C_1$-$C_6$-Alkoxygruppen, gegebenenfalls durch Halogenatome oder $C_1$-$C_4$-Alkansulfonyloxygruppen substituierte $C_2$-$C_6$-Alkanoyloxygruppen oder $C_3$-$C_6$-Alkenoyloxygruppen bedeuten, wobei mindestens einer der Reste $R_1$, $R_2$, $R_3$ oder $R_4$ kein Wasserstoffatom ist, und X für das Äquivalent eines physiologisch verträglichen Anions steht.

2. Verbindungen nach Anspruch 1,
dadurch gekennzeichnet,
daß beide Reste $R_1$ und $R_3$ jeweils in 3- oder 4-Stellung stehen und $R_2$ und $R_4$ Wasserstoff sind, oder daß der Rest $R_1$ in 3- oder 4-Stellung steht und $R_2$, $R_3$ und $R_4$ Wasserstoff sind.

3. Verbindungen nach Anspruch 1 und/oder 2,
dadurch gekennzeichnet,
daß die Reste $R_1$ und $R_3$ gleich sind und eine $C_1$-$C_4$-Alkoxygruppe, eine Hydroxygruppe, eine $C_2$-$C_6$-Alkanoyloxygruppe oder eine $C_3$-$C_6$-Alkenoyloxygruppe bedeuten und $R_2$ und $R_3$ Wasserstoff sind, oder daß $R_1$ die genannten Bedeutungen hat und $R_2$, $R_3$ und $R_4$ Wasserstoff ist.

4. Verbindungen nach Anspruch 1,
dadurch gekennzeichnet,
daß die Reste $R_1$ und $R_2$ in 3,4-Stellung stehen und $R_3$ und $R_4$ Wasserstoff sind.

5. Verbindungen nach Anspruch 4,
dadurch gekennzeichnet,
daß die Reste $R_1$ und $R_2$ gleich sind und eine $C_1$-$C_4$-Alkoxygruppe, eine Hydroxygruppe, eine $C_2$-$C_6$-Alkanoyloxygruppe oder eine $C_3$-$C_6$-Alkenoyloxygruppe bedeuten.

6. Verbindungen nach Anspruch 1,
dadurch gekennzeichnet,
daß die Reste $R_1$ und $R_2$ sowie die Reste $R_3$ und $R_4$ jeweils in 3,4-Stellung stehen.

7. Verbindungen nach Anspruch 6,
dadurch gekennzeichnet,
daß die Reste $R_1$, $R_2$, $R_3$ und $R_4$ gleich sind und eine $C_1$-$C_4$-Alkoxygruppe, eine Hydroxygruppe, eine $C_2$-$C_6$-Alkanoyloxygruppe oder eine $C_3$-$C_6$-Alkenoyloxygruppe bedeuten.

8. Verbindungen nach einem oder mehreren der vorangegangenen Ansprüche,
dadurch gekennzeichnet,
daß die Anionen der Chlorwasserstoffsäure, der Bromwasserstoffsäure, der Schwefelsäure, der Phosphorsäure, von $C_1$-$C_4$-Alkansulfonsäuren, der 1,1-Cyclobutandicarbonsäure oder der Maleinsäure für X beziehungsweise 2 X stehen können.

9. Verfahren zur Herstellung von (1,2-Diphenyl-ethylendiamin)-platin(II)-Komplexverbindungen der allgemeinen Formel

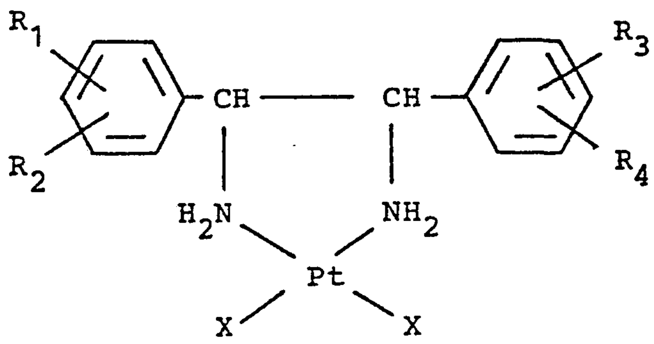

I

worin die Reste $R_1$, $R_2$, $R_3$ und $R_4$ gleich oder verschieden sind und Wasserstoff, Hydroxygruppen, $C_1$-$C_6$-Alkoxygruppen, gegebenenfalls durch Halogenatome oder $C_1$-$C_4$-Alkansulfonyloxygruppen substituierte $C_2$-$C_6$-Alkanoyloxygruppen oder $C_3$-$C_6$-Alkenoyloxygruppen bedeuten, wobei mindestens einer der Reste $R_1$, $R_2$, $R_3$ oder $R_4$ kein Wasserstoffatom ist, und X für das Äquivalent eines physiologisch verträglichen Anions steht,

dadurch gekennzeichnet,

daß man eine Tetrahalogeno-platin(II)-säure oder ein Alkali-tetrahalogeno-platin(II)-Komplexsalz mit einer Verbindung der Formel

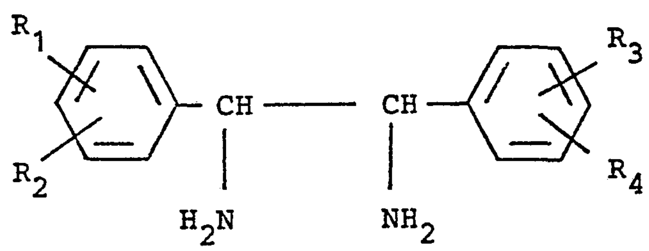

II

worin die Reste $R_1$, $R_2$, $R_3$ und $R_4$ die angegebenen Bedeutungen haben, wobei mindestens einer der Reste $R_1$, $R_2$, $R_3$ oder $R_4$ kein Wasserstoffatom ist, beziehungsweise einem Säureadditionssalz der Verbindung II umsetzt, gegebenenfalls in vorhandene freie phenolische Hydroxygruppen eine gegebenenfalls durch Halogenatome oder $C_1$-$C_4$-Alkansulfonyloxygruppen substituierte $C_2$-$C_6$-Alkanoyl- oder $C_3$-$C_6$-Alkenoylgruppe einführt und gegebenenfalls in einer Verbindung der Formel I den Rest X oder die Reste X gegen ein anderes physiologisch verträgliches Anion austauscht.

10. Arzneimittel, enthaltend eine Verbindung der allgemeinen Formel I neben üblichen Träger- und/oder Verdünnungs- beziehungsweise Hilfsstoffen.

11. Verfahren zur Herstellung eines Arzneimittels,

dadurch gekennzeichnet,

daß eine Verbindung der allgemeinen Formel I mit gebräuchlichen pharmazeutischen Trägerstoffen oder Verdünnungsmitteln beziehungsweise sonstigen Hilfsstoffen zu pharmazeutischen Zubereitungen verarbeitet beziehungsweise in eine therapeutisch anwendbare Form gebracht wird.

12. Verwendung von Verbindungen der allgemeinen Formel I zur Herstellung von Arzneimitteln.

**Claims**

1. (1,2-Diphenyl-ethylene diamine)-platinum(II)-complex compounds corresponding to the general formula

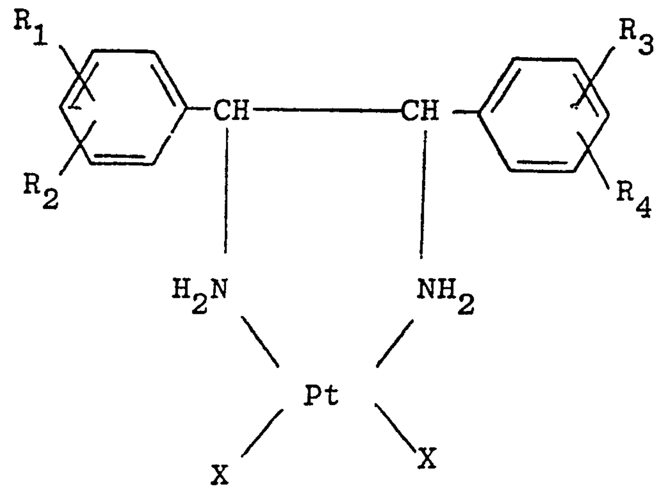

I

wherein the radicals $R_1$, $R_2$, $R_3$ and $R_4$ are the same or different and represent hydrogen hydroxy groups $C_1$-$C_6$-alkoxy groups, $C_2$-$C_6$- alkanoyloxy groups or $C_3$-$C_6$-alkenoyloxy groups optionally substituted by halogen atoms or $C_1$-$C_4$-alkane sulphonyloxy groups, wherein at least one of the radicals $R_1$, $R_2$, $R_3$ or $R_4$ is not a hydrogen atom and X representing the equivalent of a physiologically compatible anion.

2. Compounds according to claim 1, characterised in that the two radicals $R_1$ and $R_3$ each stand in the 3- or 4-position and $R_2$ and $R_4$ are hydrogen or in that the radical $R_1$ stands in the 3- or 4- position and $R_2$, $R_3$ and $R_4$ are hydrogen.

3. Compounds according to claim 1 and/or 2, characterised in that the radicals $R_1$ and $R_3$ are the same and represent a $C_1$- $C_4$- alkoxy group a hydroxy group, a $C_2$- $C_6$- alkanoyloxy group or a $C_3$- $C_6$- alkenoyloxy group and $R_2$ and $R_3$ are hydrogen, or in that $R_1$ has the meanings given above and $R_2$, $R_3$ and $R_4$ are hydrogen.

4. Compounds according to claim 1, characterised in that the radicals $R_1$ and $R_2$ stand in the 3, 4- position and $R_3$ and $R_4$ are hydrogen.

5. Compounds according to claim 4, characterised in that the radicals $R_1$ and $R_2$ are the same and represent a $C_1$-$C_4$-alkoxy group, a hydroxy group, a $C_2$-$C_6$- alkanoyloxy group or a $C_3$- $C_6$- alkenoyloxy group.

6. Compounds according to claim 1, characterised in that the radicals $R_1$ and $R_2$ as well as the radicals $R_3$ and $R_4$ each stand in the 3, 4- position.

7. Compounds according to claim 6, characterised in that the radicals $R_1$, $R_2$, $R_3$ and $R_4$ are the same and represent a $C_1$-$C_4$- alkoxy group, a hydroxy group, a $C_2$-$C_6$-alkanoyloxy group or a $C_3$-$C_6$-alkenoyloxy group.

8. Compounds according to one or more of the preceding claims, characterised in that the anions of hydrochloric acid, hydrobromic acid, sulphuric acid, phosphoric acid, of $C_1$-$C_4$-alkane sulphonic acids, 1,1-cyclobutane dicarboxylic acid or maleic acid can stand for X or 2X.

9. A process for the production of (1,2-diphenyl ethylene diamine)platinum(II) complex compounds corresponding to the general formula

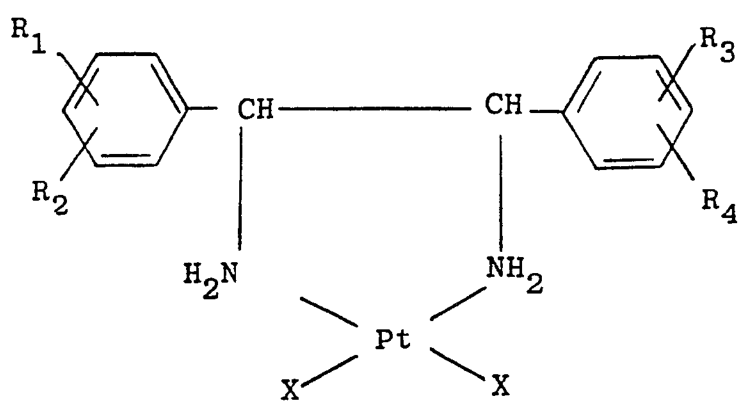

I

wherein the radicals $R_1$, $R_2$, $R_3$ and $R_3$ are the same or different and represent hydrogen hydroxy groups, $C_1$-$C_6$-alkoxy groups $C_2$-$C_6$- alkanoyloxy groups or $C_3$-$C_6$-alkenoyloxy groups optionally substituted by halogen atoms or $C_1$-$C_4$-alkane sulphonyloxy groups wherein at least one of the radicals $R_1$, $R_2$, $R_3$ or $R_4$ is not a hydrogen atom, and X represents the equivalent of a physiologically compatible anion, characterised in that

a tetrahalogeno platinum (II) acid or an alkali tetrahalogeno platinum (II) complex salt is reacted with a compound corresponding to the formula

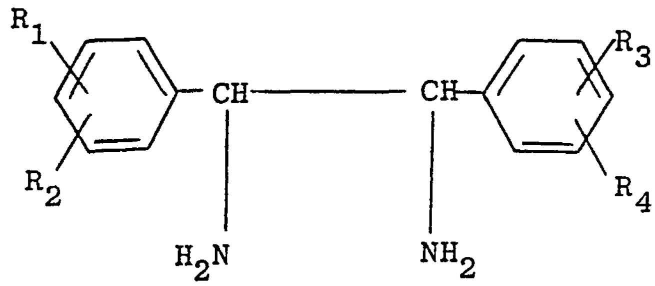

II

wherein the radicals $R_1$, $R_2$, $R_3$ and $R_4$ have the meanings given, at least one of the radicals $R_1$, $R_2$, $R_3$ or $R_4$ not being a hydrogen atom, or with an acid addition salt of compound II a $C_2$-$C_6$-alkanoyl or $C_3$-$C_6$-alkenoyl group optionally substituted by halogen atoms or $C_1$- $C_4$- alkane sulphonyloxy groups is optionally introduced into existing free phenolic hydroxy groups and the radical X or the radicals X are optionally exchanged for a different physiologically compatible anion in a compound corresponding to formula I.

10. A pharmaceutical composition containing a compound corresponding to general formula I in addition to conventional carriers and/or diluents or auxiliaries.

11. A process for the production of a pharmaceutical composition, characterised in that a compound corresponding to general formula I is processed with conventional pharmaceutical carriers or diluents or other auxiliaries to form pharmaceutical preparations or is brought into a therapeutically applicable form.

12. Use of compounds corresponding to general formula I for the production of pharmaceutical compositions.

**Revendications**

1. Complexes de (1,2-diphényl-éthylènediamine)-platine(II) de formule générale

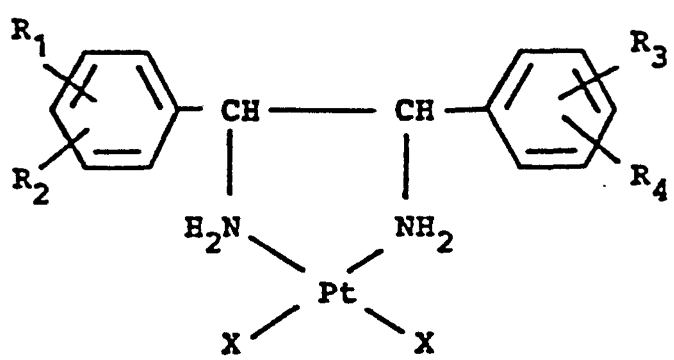

I

dans laquelle les radicaux $R_1$, $R_2$, $R_3$ et $R_4$ sont identiques ou differents et représentent des atomes d'hydrogène, des groupes hydroxy, des groupes alcoxy en $C_1$-$C_6$, des groupes $C_2$-$C_6$-alcanoyloxy ou $C_3$-$C_6$-alcénoyloxy éventuellement substitués par des atomes d'halogènes ou des groupes $C_1$-$C_4$-alcanesulfonyloxy, l'un au moins des radicaux $R_1$, $R_2$, $R_3$ ou $R_4$ n'étant pas un atome d'hydrogène, et X est mis pour l'equivalent d'un anion acceptable physiologiquement.

2. Composés selon la revendication 1, caractérisés en ce que les deux radicaux $R_1$ et $R_3$ sont respectivement en position 3 ou 4 et $R_2$ et $R_4$ sont des atomes d'hydrogène, ou en ce que le radical $R_1$ est en position 3 ou 4 et $R_2$, $R_3$ et $R_4$ sont des atomes d'hydrogène.

3. Composés selon la revendication 1 ou 2, caractérisés en ce que les radicaux $R_1$ et $R_3$ sont identiques et représentent chacun un groupe alcoxy en $C_1$-$C_4$, un groupe hydroxy, un groupe $C_2$-$C_6$-alcanoyloxy ou un groupe $C_3$-$C_6$-alcénoyloxy et $R_2$ et $R_3$ sont des atomes d'hydrogène, ou en ce que $R_1$ a les significations données ci-dessus et $R_2$, $R_3$ et $R_4$ sont des atomes d'hydrogène.

4. Composés selon la revendication 1, caractérisés en ce que les radicaux $R_1$ et $R_2$ sont en position 3, 4 et $R_3$ et $R_4$ sont des atomes d'hydregène.

5. Composés selon la revendication 4, caractérisés en ce que les radicaux $R_1$ et $R_2$ sont identiques et représentent un groupe alcoxy en $C_1$-$C_4$, un groupe hydroxy, un groupe $C_2$-$C_6$-alcanoyloxy ou un groupe $C_3$-$C_6$-alcénoyloxy.

6. Composés selon la revendication 1, caractérisés en ce que les radicaux $R_1$ et $R_2$, ainsi que les radicaux $R_3$ et $R_4$ sont respectivement en position 3, 4.

7. Composés selon la revendication 6, caractérisés en ce que les radicaux $R_1$, $R_2$, $R_3$ et $R_4$, sont identiques et représentent chacun un groupe alcoxy en $C_1$-$C_4$, un groupe hydroxy, un groupe $C_2$-$C_6$-alcanoyloxy ou un groupe $C_3$-$C_6$-alcénoyloxy.

8. Composés selon l'une quelconque des revendications 1 à 7, caractérisés en ce que les anions de l'acide chlorhydrique, de l'acide bromhydrique, de l'acide sulfurique, de l'acide phosphorique, d'acides $C_1$-$C_4$-alcanesulfoniques, de l'acide 1,1-cyclobutanedicarboxylique ou de l'acide maléique peuvent être mis pour X ou pour 2 X.

9. Procédé de préparation de complexes de (1,2-diphényl-éthylénediamine)-platine(II) de formule générale

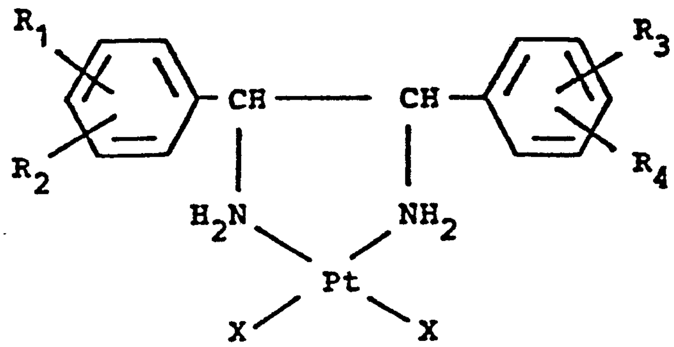

I

dans laquelle les radicaux $R_1$, $R_2$, $R_3$ et $R_4$ sont identiques ou différents et représentent des atomes d'hydrogène, des groupes hydroxy, des groupes alcoxy en $C_1$-$C_6$, des groupes $C_2$-$C_6$-alcanoyloxy ou $C_3$-$C_6$-alcénoyloxy éventuellement substitués par des atomes d'halogènes ou des groupes $C_1$-$C_4$-alcanesulfonyloxy, l'un au moins des radicaux $R_1$, $R_2$, $R_3$ ou $R_4$ n'étant pas un atome d'hydrogène, et X est mis pour l'équivalent d'un anion acceptable physiologiquement, caractérisé en ce qu'on fait réagir un acide tétrahalogénoplatineux ou un sel de complexe d'alcali-tétrahalogénoplatine(II) avec un composé de formule

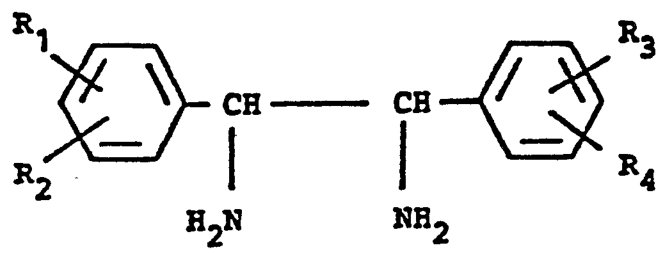

II

dans laquelle les radicaux $R_1$, $R_2$, $R_3$ et $R_4$ ont les significations données ci-dessus, l'un au moins des radicaux $R_1$, $R_2$, $R_3$ ou $R_4$ n'étant pas un atome d'hydrogène, ou avec un sel d'addition d'acide du composé II, on introduit le cas échéant, dans des groupes hydroxy phénoliques libres présents, un groupe $C_2$-$C_6$-alcanoyle ou $C_3$-$C_6$-alcénoyle éventuellement substitué par des atomes d'halogènes ou des groupes $C_1$-$C_4$-alcanesulfonyloxy et, le cas échéant, on échange, dans un composé de formule I, le radical X ou les radicaux X contre un autre anion acceptable physiologiquement.

10. Médicament contenant, outre des excipients et/ou diluants ou adjuvants usuels, un composé de formule générale I.

11. Procéde de préparation d'un médicament, caractérise en ce qu'un composé de formule genérale I est travaillé avec des excipients, diluants ou autres adjuvants pharmaceutiques usuels pour donner des préparations pharmaceutiques ou est mis sous une forme applicable thérapeutiquement.

12. Utilisation de composés de formule générale I pour la préparation de médicaments.